# EUROPEAN PATENT SPECIFICATION

(11) **EP 1 493 736 B1**
(45) Date of publication and mention of the grant of the patent: **19.07.2006**
(21) Application number: 04017660.4
(22) Date of filing: 06.08.1998
(51) Int. Cl.: C07D 293/06, C07H 15/26, C07H 17/00

(54) **Prodrugs and conjugates of selenium-containing compounds and methods of use thereof**
Prodrugs und Konjugate von Selenoenthaltenden Verbindungen und ihre Verwendung
Promédicaments et conjugués de composés contenant du sélénium et leur utilisation

(30) Priority: 07.08.1997 US 55019 P
(43) Date of publication of application: 05.01.2005
(62) Divisional of application: 98940791.1
(73) Proprietor: University of Utah Research Foundation, Salt Lake City, Utah 84108 (US)
(72) Inventor: Roberts, Jeannette, C., Salt Lake City, Utah 84108 (US); Short, Megan, D., Salt Lake City, Utah 84102 (US); Dominick, Pamela, K., Salt Lake City, Utah 84109 (US); Cassidy, Pamela, B., Salt Lake City, Utah 84102 (US); Wilmore, Britta, H., Salt Lake City, Utah 84115 (US)
(74) Representative: Gowshall, Jonathan Vallance

(56) References cited:
- DRAGUET, C. ET AL: "Selenazolidines. I. Synthesis of selenazolidine, N-alkylselenazolidine, and their 2-substituted derivatives" BULLETIN DES SOCIETES CHIMIQUES BELGES , 81(3-4), 279-87 CODEN: BSCBAG; ISSN: 0037-9646, 1972, XP009036104

## Description

### FIELD OF THE INVENTION

The present invention relates to selenium- containing compounds and methods for using these compounds to protect mammals from toxic insults. More specifically, the present invention relates to prodrugs and conjugates of selenol-containing compounds, such as selenocysteamine, and the Walter Reed (WR) compounds.

### BACKGROUND OF THE INVENTION

### Technical Background

Thiol- or selenol-containing compounds, e.g., cysteine, cysteamine, glutathione, selenocysteine, selenocysteamine, and the WR compounds, are known protective and preventive agents. Potential protective or preventive uses of such agents are widespread, as in reducing the unwanted side effects of chemo- or radiotherapy of cancer, improving cardiovascular function, preventing mutagenesis, preventing the initiation and/or progression of cancer, reducing toxic consequences of planned or unplanned radiation or chemical exposures, slowing the aging process, and preventing cataract formation. New evidence also links these compounds to altered gene expression and enhanced-cellular repair processes.

The activity of these thiol- or selenol-containing compounds is mainly due to the sulfur or selenium atom participating in nucleophilic attack on toxic electrophiles, scavenging free radicals, effecting repair of damaged targets through hydrogen atom donation, altering the redox status of the cell, or affecting gene transcription or protein function.

For example, the reduced form of glutathione (Glu-Cys-Gly), a naturally occurring tripeptide with a free sulfhydryl group (SH), serves as a sulfhydryl buffer that maintains the cysteine residues of hemoglobin and other proteins in a reduced state. Glutathione also plays a key role in detoxifying the body by reacting with both endogenous and exogenous compounds, such as hydrogen peroxide and other peroxides.

Evidence suggests that glutathione is useful at protecting the body from the harmful side effects of radiation and chemotherapy that often accompany cancer treatment. Cyclophosphamide (CTX), for example, is a widely used antitumor agent whose clinical utility is limited by its bladder toxicity. During CTX metabolism in the body, a compound, acrolein, is released. Acrolein is thought to be responsible for the urotoxicity of CTX. Glutathione has been implicated in CTX detoxification by conjugating to acrolein.

It has been of significant interest in the art, therefore, to increase glutathione synthesis especially during periods of toxic insults. L- cysteine, a reactant in normal glutathione biosynthesis, is known to increase the synthesis of endogenous glutathione. To date, a significant challenge in the art has been to provide L-cysteine to cells at sufficiently high levels to drive glutathione biosynthesis. As disclosed, for example, in United States Patent 4,868,114 to Nagasawa et al., prodrugs of L-cysteine (i.e., chemical compounds converted to L-cysteine in the cell), such as RibCys, can be used by the cell to drive glutathione biosynthesis shown below.

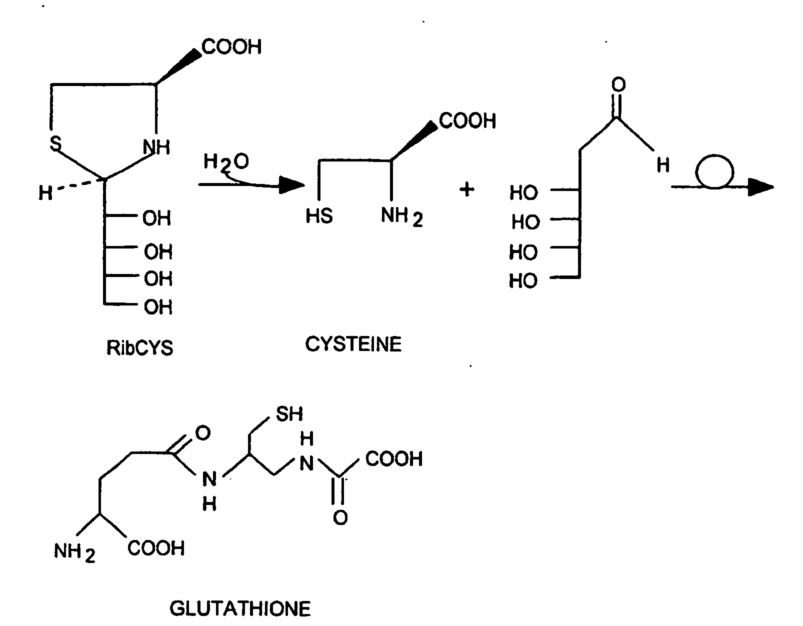

These prodrugs have been shown to offer good protection against a variety of toxic insults. However, the initial prodrugs are highly water soluble and are rapidly excreted by the body.

WR compounds are also of significant interest in the art. Over 4400 WR compounds were prepared and tested at the Walter Reed Army Hospital after World War II in an effort to develop radioprotective compounds that might be employed by military personnel during a nuclear encounter. The single agent with the greatest potential that arose from that extensive effort was WR-2721. which is converted to WR-1065 by enzymatic cleavage. These compounds have several shortcomings, however, including that they possess noteworthy toxicity and little oral activity, greatly reducing their clinical utility.

Finally, selenocysteines are of significant interest in the art for their antioxidant and anticancer properties. In fact. selenium has received significant attention for its ability to inhibit or delay the onset of AIDS caused by HIV infection. Selenium is also a cofactor of glutathione peroxidase, an enzyme which has been implicated in many detoxifying processes.

Selenium is an essential mineral that is critical to the normal functioning of many species, including humans. It also has demonstrated activity as a cancer chemopreventive agent. Selenium-containing compounds appear to have especially high preventive activity against the initiation phase of colorectal cancer, although its chemoprotective ability has been extended to cancers in many organs, caused by a variety of carcinogens.

To achieve this chemopreventive activity, levels of selenium at least five-fold greater than that required for normal nutritional status appear to be necessary. In addition, selenium must be given continuously for maximum inhibition. Unfortunately, selenium is also known for its profound toxicity, making selenium supplementation a distinct challenge.

Current selenium supplements rely on inorganic forms, such as sodium selenite (Na₂SeO₃) or sodium selenate (Na₂SeO₄). While these forms have some value, they are considered more toxic than necessary, and are unlikely to be useful in cancer chemo-prevention. Several organoselenium compounds, which appear to be less toxic in general than the inorganic forms. have been proposed for in vivo use, but the full potential of this strategy has not yet been realized. In general, however, it is very clear that the chemical form in which selenium is introduced consistently shows a marked influence on biological outcomes. including cancer chemoprevention and toxicity.

Selenocysteine is an organic form that is present in the body and is now recognized as the 21st amino acid used in protein synthesis. While it represents a valuable biochemical form, selenocysteine is chemically unstable and difficult to handle, which has undoubtedly deterred its study and use. In addition, even though it possesses greatly reduced inherent toxicity, it still may be too toxic at chemopreventive doses to the therapeutically useful. Accordingly, prodrug forms of selenocysteine that possess reduced inherent toxicity and improved physicochemical properties would be desirable.

Bull. Soc. Chim. Belges, 81 (1972) 279-287 discloses a general method of synthesis of a selenazolidine and selenazolidines substituted in positions 2 and 3.

### Objects of the Invention

It is, therefore, an object of the invention to provide prodrugs and conjugates of selenol-containing compounds, such as selenocysteamine, and the WR compounds.

Another object of the invention is to provide such selenol-containing compounds displaying reduced toxicity and increased clinical utility.

Another object of the invention is to provide such selenol-containing compounds with increased lipophilicity that can target a specific organ or region of the body.

Another object of the invention is to provide such selenol-containing compounds that can be conjugated to antioxidants, such as vitamin C and E, thus maximizing the effects by providing different agents that work by complementary mechanisms.

Further objects of the invention will become evident in the description below.

### BRIEF SUMMARY OF THE INVENTION

The present invention is directed to novel prodrugs and conjugates of selenol-containing compounds, including selenocysteamine, and the WR compounds. Potential protective or preventive uses of such agents are widespread, as in reducing the unwanted side effects of chemo- or radiotherapy of cancer, improving cardiovascular function, preventing mutagenesis, preventing the initiation and/or progression of cancer, reducing toxic consequences of planned or unplanned radiation or chemical exposures, slowing the aging process, preventing cataract formation, etc.

Prodrugs are inactive forms of a parent drug that have been created to overcome one or more barriers to their effective use. In the present invention, prodrugs have been designed to overcome the chemical instability and/or possible toxicity barriers that exist with the parent drug.

A first embodiment relates to the design, synthesis, and evaluation of novel prodrugs that are derivatives of selenocysteamines, such as of WR compounds, particularly WR-1065. The prodrug strategy is similar to that employed for L-cysteine, using a protecting group R'. R is typically a sugar, such as ribose. The modified WR prodrugs have numerous uses including protection against the side effects of radiation and chemotherapy, radiation and chemical induced mutations, such as from exposure to radiation during a nuclear accident or chemical spill, and even spontaneous mutations which are the cause of most cancers.

These prodrugs can be described by the formulas; 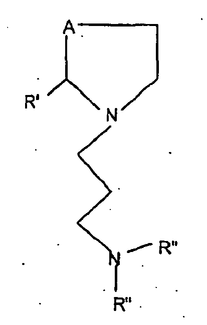 where A is selenium, R' is derived from a sugar and has the formula (CHOH)ₙCH₂OH, where n is 1 to 5. R' may also be hydrogen, an alkyl or aryl group, such as methyl, ethyl, benzyl, carboxyl, polyhydroxyalkyl, or phenyl, or may also be =O. The R" groups may be the same or different and may be alkyl, alkoxy, carboxy, such as acetyl, methyl or ethyl.

These novel selenol-containing compounds overcome several problems facing the art, including toxicity, water-solubility, and lack of target specificity. First, the protective or preventive activity and clinical utility will be greatly enhanced by converting the cysteine, cysteamine, glutathione, selenocysteine, selenocysteamine, and WR compounds, selenazolidone prodrug forms. These prodrugs provide a slow release form of the selenol-amine, which greatly reduces observed toxicity (with related compounds), but provides the active agent after enzymatic or non-enzymatic biotransformation

In another embodiment, the invention relates to the design, synthesis, and evaluation of novel covalent conjugates of selenolamines and antioxidant vitamins, e.g., Vitamin E and Vitamin C. These compounds include conjugates of any of the prodrug compounds of the invention defined above conjugated with Vitamin C or Vitamin E.

These compounds are effective because protective or preventive treatment of toxic insult will be far more effective if selenol-containing compounds are delivered together with antioxidants such as vitamin C and E which also play a protective and preventative action in the body. The complementary mechanisms of these compounds would increase the overall effectiveness of treatment.

In yet another embodiment, the invention relates to the design, synthesis, and evaluation of novel prodrugs which have been modified with ester or amine groups at the carboxylic acid position. These can be described by formula; 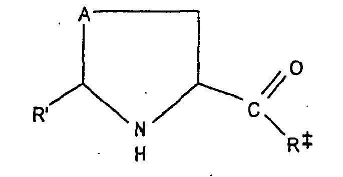 where A is selenium, and R' is derived from a sugar and has the formula (CHOH)ₙCH₂OH, where n is 1 to 5. R' may also be an alkyl or aryl group, such as methyl, ethyl, benzyl, carboxyl, or phenyl, or may also be =O. 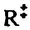 is an alkoxy, such as -OR¹ where R¹ is ethyl, methyl. 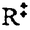 may also be an amine group (-NR⁺₂) where the R⁺ groups are the same or different and hydrogen or an alkyl group, such as methyl.

Yet another embodiment of the invention is the condensation product of a selenolamine and a carbonyl donor characterized by the formula: 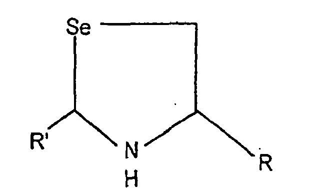 where R is COOH (prodrug of L-selenocysteine). R' is derived from a sugar and has the structure (CHOH)ₙCH₂OH; and where n is 1 to 5; an alkyl or aryl group, such as methyl, ethyl, benzyl, phenyl or carboxyl' or=O

According to the present invention, there is provided a prodrug of the formula; Se 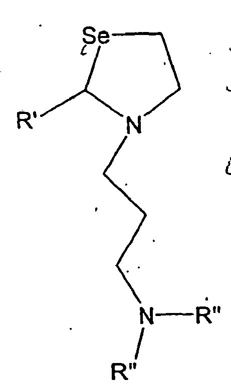 where R' is a sugar that is of the formula (CHOH)ₙCH₂OH, where n is 1 to 5, or R' is hydrogen, an alkyl or aryl group, or
R' is=O, and
R" groups are the same or different and may be hydrogen, alkyl, alkoxy, or carboxy.

Preferably, R' is methyl, ethyl, benzyl, carboxyl, phenyl, or polyhydroxyakyl.

Advantageously, R" is hydrogen, acetyl, methyl, or ethyl.

The present invention also provides a prodrug of the formula

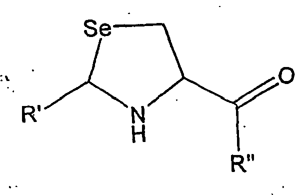

Where R' is a sugar and is of the formula (CHOH)ₙ CH₂OH, where n is 1 to 5, or R' is an alkyl or aryl group, or
R' is=O, and
R" is an alkoxy or an amine group.

Preferably, R" is OR¹, where R¹ is ethyl or methyl.

Conveniently, R' is methyl, ethyl, benzyl, carboxyl, or phenyl.

Advantageously, R" is-NR², wherein the R² groups are the same or different and are hydrogen or akyl.

Most preferably, at least one R" is methyl.

The present invention further provides a prodrug of the formula 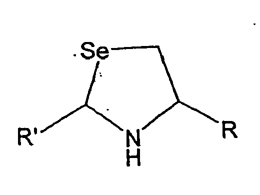 R is COOH and
R' is a sugar of the formula (CHOH)ₙCH₂OH, wherein n is 1 to 5, or
R' is an alkyl or aryl group, or
R' is=O

Preferably, R' is methyl, ethyl, benzyl, carboxyl, or phenyl.

In a further embodiment, the present invention provides a conjugate of an antioxidant vitamin and a prodrug of the present invention.

Preferably, the antioxidant is vitamin C or vitamin E.

### DETAILED DESCRIPTION

### I. Covalent Conjugates of Thiol- or Selenol-amines and Antioxidant Vitamins

### 1. Agent Design

The present invention focuses on the antioxidant vitamins C and E, and the selenol agents, selenocysteine, and selenocysteamine. It will be appreciated by those skilled in the art that other antioxidants can be conjugated to these selenol-containing compounds.

### 2. Chemical Synthesis

The schemes below summarize potential approaches using cysteamine for illustrative purposes. Many permutations are available.

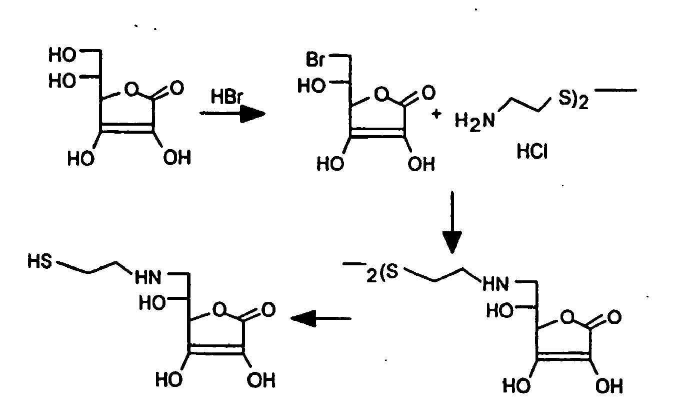

### II Modified Prodrugs L-Selenochysteine

### 1. Agent Design

These prodrugs possess a modified carboxyl group compared to unmodified prodrugs of L-selenocysteine. The purpose of the modification is to reduce the hydrophilicity of the prodrugs and improve their cellular uptake and retention in the body. The modifications include converting the carboxyl group to an ester or amide functionality.

### 2. Chemical Synthesis

Ester prodrugs were prepared beginning with commercially available L-cysteine methyl or ethyl ester. The ester is combined with an equimolar amount of carbonyl donor, i.e., acetaldehyde, the aldose monosaccharide, D-ribose, or phenyl chloroformate. The amide prodrugs were prepared by the initial synthesis of L-cysteine amides (not commercially available) from L-cysteine and the appropriate amine, such as ammonia, methylamine, or dimethylamine. The synthesized L-cysteine amides were then reacted with an equimolar amount of carbonyl donor, i.e., acetaldehyde, the aldose monosaccharide, D-ribose, or phenyl chloroformate.

Modified prodrugs of L-selenocysteine can be constructed in an identical fashion. However, L-selenocysteine methyl or ethyl ester are prepared by the esterification of L-selenocysteine with methanol or ethanol because these compounds are not commercially available.

For example, the reaction of L-cysteine ethyl ester and D-ribose may be as follows;

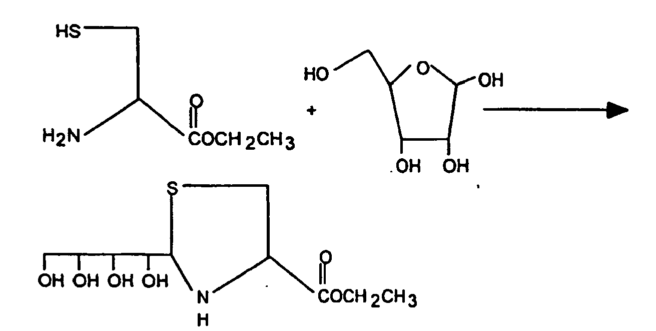

### III. Selenazolidines: Modified Prodrugs of Selenocysteine and Selenocysteamine

### 1. Agent Design

Current selenium supplements rely on inorganic forms. While these forms have some value, they are considered more toxic than necessary, and are unlikely to be useful in cancer chemoprevention or in AIDS supplementation. Several organoselenium compounds, which appear to be less toxic in general than the inorganic forms, have been proposed for in vivo use, but the full potential of this strategy has not yet been realized. In general, however, it is very clear that the chemical form in which selenium is introduced consistently shows a marked influence on biological outcomes. Selenocysteine is an organic form that is present in the body and is now recognized as the 21 st amino acid used in protein synthesis. Due to its differential metabolism, it represents the biochemically superior form in which to supply the body with selenium. Unfortunately, selenocysteine is chemically unstable and difficult to handle. Therefore, prodrug forms of the amino acid have been designed which represent chemically superior forms. Similar arguments hold for selenocysteamine as well.

### 2. Chemical Synthesis

Selenocysteine/selenocysteamine prodrugs can be synthesized by the chemical condensation of the selenolamine with a carbonyl donor. Alkyl or alkyl aldehydes or ketones can be used, including simply donors such as acetaldehyde or benzaldehyde, or aldose or ketose mono- or di-saccharides. In addition, carbonyl donors such as phenyl chloroformate can be used to produce 2-oxo derivatives.

For example, the reaction of L-selenocysteine and phenyl chloroformate is illustrated.

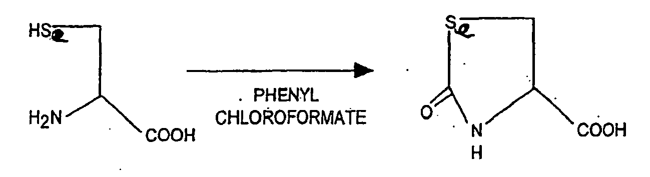

### Preferred Features of the Disclosure

1. A prodrug of the formula; 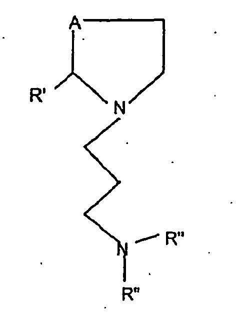 where A is selenium,
   R' is derived from a sugar and R' has the formula (CHOH)ₙCH₂OH, where n is 1 to 5, or
   R' is an alkyl or aryl group, or
   R' is =O, and
   the R" groups may be the same or different and may be hydrogen, alkyl, alkoxy, or carboxy.
2. A prodrug of clause 3 wherein R' is methyl, ethyl, benzyl, carboxyl, phenyl, polyhydroxyalkyl.
3. A prodrug of clause 3 wherein R" is hydrogen, acetyl, methyl or ethyl.
4. A conjugate of an antioxidant vitamin and a thiolamine or selenolamine.
5. A conjugate as in clause 6 wherein the antioxidant vitamin is Vitamin C or Vitamin E.
6. A conjugate as in clause 6 wherein the thiolamine or selenolamine selected from or a derivative of the group comprising cysteine, cystine, cysteamine, cystamine; glutathione, selenocysteine, selenocysteamine, selenocystine, selenocystamine; WR-1065, and WR-33278.
7. A prodrug of the formula; 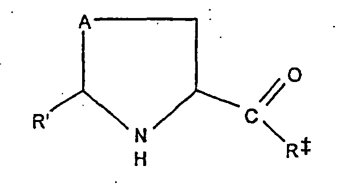 where A is selenium, and
   R' is derived from a sugar and R' has the formula (CHOH)ₙCH₂OH, where n is 1 to 5, or
   R' is also be an alkyl or aryl group, or
   R' is =O, and
   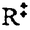 is an alkoxy, or an amine group.
8. A prodrug as in clause 9 wherein 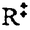 is -OR¹ where R¹ is ethyl, or methyl.
9. A prodrug as in clause 9 wherein R' is methyl, ethyl, benzyl, carboxyl, or phenyl.
10. A prodrug as in clause 9 wherein 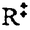 is -NR⁺₂, wherein the R⁺ groups are the same or different and are hydrogen or alkyl.
11. A prodrug as in clause 12 wherein at least one R⁺ is methyl.
12. A prodrug of the formula: 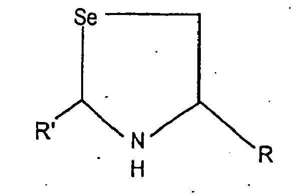 R is COOH, and
   R' is derived from a sugar and R' has the formula (CHOH)ₙCH₂OH, where n is 1 to 5, or
   R' is an alkyl or aryl group, or
   R' is =O.
13. A prodrug of clause 14 wherein R' is methyl, ethyl, benzyl, carboxyl, or phenyl.

## Claims

1. A prodrug of the formula; 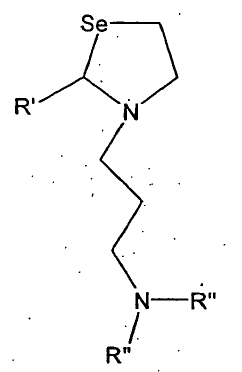 where R' is a sugar that is of the formula (CHOH)ₙCH₂OH, where n is 1 to 5, or R' is hydrogen, an alkyl or aryl group, or
R' is =O, and
R" groups are the same or different and may be hydrogen, alkyl, alkoxy, or carboxy.

2. The prodrug of claim 1, wherein R' is methyl, ethyl, benzyl, carboxyl, phenyl, or polyhydroxyalkyl.

3. The prodrug of claim 1, wherein R" is hydrogen, acetyl, methyl, or ethyl.

4. A prodrug of the formula; 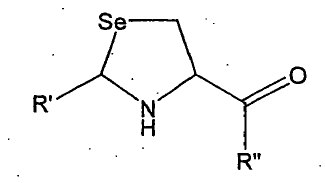 where R' is a sugar and is of the formula (CHOH)ₙCH₂OH, where n is 1 to 5, or R' is an alkyl or aryl group, or
R' is =O, and
R" is an alkoxy or an amine group.

5. The prodrug of claim 4, wherein R" is -OR¹, where R¹ is ethyl or methyl.

6. The prodrug of claim 4, wherein R' is methyl, ethyl, benzyl, carboxyl, or phenyl.

7. The prodrug of claim 4, wherein R" is NR², wherein the R² groups are the same or different and are hydrogen or alkyl.

8. The prodrug of claim 7, wherein at least one R² is methyl

9. A prodrug of the formula: 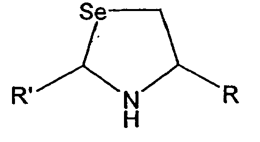 R is COOH and
R' is a sugar of the formula (CHOH)ₙCH₂OH, where n is 1 to 5, or
R' is an alkyl or aryl group, or
R' is=O.

10. The prodrug of claim 9, wherein R' is methyl, ethyl, benzyl, carboxyl, or phenyl.

11. A conjugate of an antioxidant vitamin and a prodrug of claims 1-10.

12. The conjugate of claim 11, wherein the antioxidant is Vitamin C or Vitamin E.

13. A prodrug or conjugate of claims 1-12 for use as a medicament for toxic insult.

14. A prodrug or conjugate of claims 1-12 as a medicament for (1) reducing unwanted side effects of chemo- or radiotherapy of cancer, (2) improving cardiovascular function, (3) preventing mutagenesis, (4) preventing the initiation and/or progression of cancer, (5) reducing toxic consequences of planned or unplanned radiation or chemical exposures, (6) slowing the aging process, or (7) preventing cataract formation.

15. Use of a prodrug or conjugate of claims 1-12 for the manufacture of a medicament for toxic insult.

16. Use of a prodrug or conjugate of claims 1-12 for the manufacture of a medicament for (1) reducing unwanted side effects of chemo- or radiotherapy of cancer, (2) improving cardiovascular function, (3) preventing mutagenesis, (4) preventing the initiation and/or progression of cancer, (5) reducing toxic consequences of planned or unplanned radiation or chemical exposures, (6) slowing the aging process, or (7) preventing cataract formation.

## Patentansprüche

1. Prodrug der Formel: 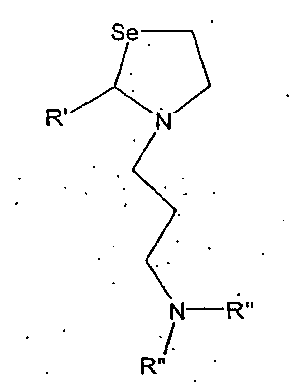 worin R' ein Zucker ist, der die Formel (CHOH)ₙCH₂OH besitzt, worin n 1 bis 5 ist, oder
R' Wasserstoff, eine Alkyl- oder Arylgruppe ist oder
R' =O ist und
R"-Gruppen identisch oder verschieden sind und Wasserstoff, Alkyl, Alkoxy oder Carboxy sein können.

2. Prodrug nach Anspruch 1, **dadurch gekennzeichnet, daß** R' Methyl, Ethyl, Benzyl, Carboxyl, Phenyl oder Polyhydroxyalkyl ist.

3. Prodrug nach Anspruch 1, **dadurch gekennzeichnet, daß** R" Wasserstoff, Acetyl, Methyl oder Ethyl ist.

4. Prodrug der Formel: 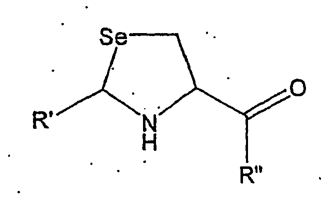 worin R' ein Zucker ist und die Formel (CHOH)ₙCH₂OH besitzt, worin n 1 bis 5 ist, oder
R' eine Alkyl- oder Arylgruppe ist oder
R' =O ist und
R" eine Alkoxy- oder eine Amingruppe ist.

5. Prodrug nach Anspruch 4, **dadurch gekennzeichnet, daß** R" -OR¹ ist, worin R¹ Ethyl oder Methyl ist.

6. Prodrug nach Anspruch 4, **dadurch gekennzeichnet daß** R' Methyl, Ethyl, Benzyl, Carboxyl oder Phenyl ist.

7. Prodrug nach Anspruch 4, **dadurch gekennzeichnet, daß** R" -NR² ist, worin die R²⁻Gruppen identisch oder verschieden sind und Wasserstoff oder Alkyl sind.

8. Prodrug nach Anspruch 7, **dadurch gekennzeichnet, daß** wenigstens ein R² Methyl ist.

9. Prodrug der Formel: 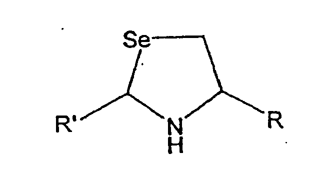 worin R COOH ist und
R' ein Zucker der Formel (CHOH)ₙCH₂OH ist, worin n 1 bis 5 ist, oder
R' eine Alkyl- oder Arylgruppe ist oder
R' =O ist.

10. Prodrug nach Anspruch 9, **dadurch gekennzeichnet, daß** R' Methyl, Ethyl, Benzyl, Carboxyl oder Phenyl ist.

11. Konjugat aus einem oxidationsverhindernden Vitamin und dem Prodrug nach den Ansprüchen 1 bis 10.

12. Konjugat nach Anspruch 11, **dadurch gekennzeichnet, daß** das Antioxidationsmittel Vitamin C oder Vitamin E ist.

13. Prodrug oder Konjugat nach den Ansprüchen 1 bis 12 zur Verwendung als ein Arzneimittel für toxischen Insult.

14. Prodrug oder Konjugat nach den Ansprüchen 1 bis 12 als ein Arzneimittel zur (1) Verringerung unerwünschter Nebenwirkungen der Chemo- oder Radiotherapie von Krebs, (2) Verbesserung der kardiovaskulären Funktion, (3) Verhinderung der Mutagenese, (4) Verhinderung der Initiation und/oder Progression von Krebs, (5) Verringerung toxischer Folgen von geplanter oder nicht-geplanter Bestrahlung oder Chemikalieneinwirkungen, (6) Verlangsamung des Alterungsprozesses oder (7) Verhinderung der Kataraktbildung.

15. Verwendung eines Prodrugs oder Konjugats nach den Ansprüchen 1 bis 12 zur Herstellung eines Arzneimittels für toxischen Insult.

16. Verwendung eines Prodrugs oder Konjugats nach den Ansprüchen 1 bis 12 zur Herstellung eines Medikaments zur (1) Verringerung unerwünschter Nebenwirkungen der Chemo- oder Radiotherapie von Krebs, (2) Verbesserung der kardiovaskulären Funktion, (3) Verhinderung der Mutagenese, (4) Verhinderung der Initiation und/oder Progression von Krebs, (5) Verringerung toxischer Folgen von geplanter oder nicht-geplanter Bestrahlung oder Chemikalieneinwirkungen, (6) Verlangsamung des Alterungsprozesses oder (7) Verhinderung der Kataraktbildung.

## Revendications

1. Promédicament de la formule 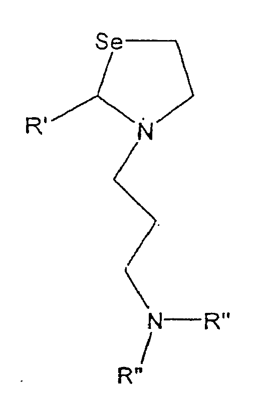 où R' est un sucre qui a la formule (CHOH)ₙCH₂OH, où n est 1 à 5, ou R' est un hydrogène, un groupe alkyle ou aryle, ou
R' est = 0, et
des groupes R" sont les mêmes ou différents et peuvent être hydrogène, alkyle, alcoxy ou carboxy.

2. Promédicament de la revendication 1, dans lequel R' est un méthyle, éthyle, benzyle, carboxyle, phényle ou polyhydroxyalkyle.

3. Promédicament de la revendication 1, dans lequel R" est un hydrogène, acétyle, méthyle ou éthyle.

4. Promédicament de la formule 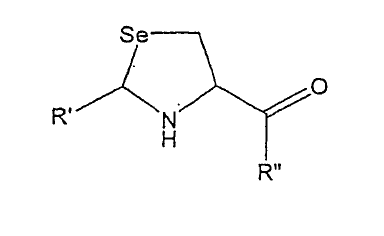 où R' est un sucre et a la formule (CHOH)ₙCH₂OH, où n est 1 à 5, ou
R' est un groupe alkyle ou aryle, ou
R' est = 0, et
R" est un groupe alcoxy ou amine.

5. Promédicament de la revendication 4, dans lequel R" est -OR¹, où R' est un éthyle ou méthyle.

6. Promédicament de la revendication 4, dans lequel R' est un méthyle, éthyle, benzyle, carboxyle ou phényle.

7. Promédicament de la revendication 4, dans lequel R" est -NR², dans lequel les groupes R² sont les mêmes ou différents et sont hydrogène ou alkyle.

8. Promédicament de la revendication 7, dans lequel au moins un R² est un méthyle.

9. Promédicament de la formule 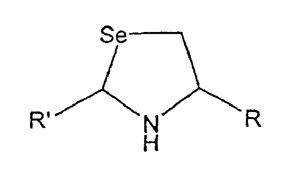 R est COOH, et
R' est un sucre de la formule (CHOH)ₙCH₂OH, où n est 1 à 5, ou
R' est un groupe alkyle ou aryle, ou
R' est = O.

10. Promédicament de la revendication 9, dans lequel R' est un méthyle, éthyle, benzyle, carboxyle ou phényle.

11. Conjugué d'une vitamine antioxydante et d'un promédicament des revendications 1-10.

12. Conjugué de la revendication 11, dans lequel l'antioxydant est la vitamine C ou la vitamine E.

13. Promédicament ou conjugué des revendications 1-12 pour une utilisation en tant que médicament pour choc toxique.

14. Promédicament ou conjugué des revendication 1-12 en tant que médicament pour (1) réduire les effets secondaires non voulus de la chimio- ou radiothérapie du cancer, (2) améliorer la fonction cardiovasculaire, (3) empêcher la mutagenèse, (4) empêcher le début et/ou la progression de cancer, (5) réduire les conséquences toxiques d'expositions chimiques ou aux radiations prévues ou imprévues, (6) ralentir le processus de vieillissement ou (7) empêcher la formation de la cataracte.

15. Utilisation d'un promédicament ou conjugué des revendications 1-12 pour la fabrication d'un médicament pour un choc toxique.

16. Utilisation d'un promédicament ou conjugué des revendications 1-12 pour la fabrication d'un médicament pour (1) réduire les effets secondaires non voulus de chimio- ou radiothérapie du cancer, (2) améliorer la fonction cardiovasculaire, (3) empêcher la mutagenèse, (4) empêcher le début et/ou la progression de cancer, (5) réduire les conséquences toxiques d'expositions chimiques ou aux radiations prévues ou imprévues, (6) ralentir le processus de vieillissement ou (7) empêcher la formation de la cataracte.
